# EUROPEAN PATENT APPLICATION

(11) **EP 3 859 005 A1**
(43) Date of publication of application: **04.08.2021**
(21) Application number: 19865332.1
(22) Date of filing: 23.08.2019
(51) Int. Cl.: C12Q 1/02, G01N 33/68

(54) **CARDIOTOXICITY ASSESSMENT METHOD**

(30) Priority: 28.09.2018 JP 2018185533
(71) Applicant: Zeon Corporation, Tokyo 100-8246 (JP)
(72) Inventor: ADACHI ,Satoru, Tokyo 100-8246 (JP); SEKINO ,Yuko, Tokyo 100-8246 (JP)
(74) Representative: Ehlich, Eva Susanne
(86) International application number: PCT/JP2019/033022
(87) International publication number: WO 2020/066396

(57) **Abstract**

Provided is a cardiotoxicity assessment method which comprises: seeding cardiomyocytes in a culture container together with a culture medium; adding a drug to the culture medium in the culture container to bring the drug into contact with the cardiomyocytes; and measuring a heart disease biomarker secreted from the cardiomyocytes to assess cardiotoxicity of the drug, wherein at least a culture surface of the culture container is composed of an alicyclic structure-containing polymer, and the culture surface has a surface free energy of 30 to 37 mN/m.

## Description

### TECHNICAL FIELD

The present disclosure relates to methods of assessing drug-induced cardiotoxicity for predicting side effects and determining doses to humans prior to clinical trials for new drugs, and in particular to methods for assessing cardiotoxicity by measuring a heart disease biomarker secreted from cardiomyocytes due to their contact with a drug.

### BACKGROUND

One method of diagnosing heart disease involves measuring a heart disease biomarker secreted from cardiomyocytes. PTL 1 teaches a method of accurately measuring blood BNP levels with the use of a BNP measurement standard that is close to real samples. PTL 2 teaches using in vitro differentiated cardiomyocytes for drug screening to assess drug efficacy and toxicity.

### CITATION LIST

### Patent Literature

PTL 1: JP2014032062A
PTL 2: JP2007537429A

### SUMMARY

### (Technical Problem)

However, no studies have been made so far to increase the measurement reproducibility by regulating the material and characteristics of containers used for biomarker measurements.

The present disclosure has been made in view of the circumstance described above and an object of the present disclosure is to provide a cardiotoxicity assessment method which measures a heart disease biomarker with high accuracy.

### (Solution to Problem)

The inventors have established that the use of a culture container which is composed of an alicyclic structure-containing polymer and whose culture surface has a surface free energy that falls within a specific range enables highly reproducible measurement of a cell-secreted biomarker. The inventors have thus completed the present disclosure.

Surface free energy herein is a criterion determined by measuring how much ink or other material adheres to the plastic or metal surface. It is expressed in units of mN/m and higher surface free energy values mean higher adhesion.

The present disclosure has been made based on the above findings and aims to advantageously solve the problem set forth above. An aspect of the present disclosure is directed to a cardiotoxicity assessment method comprising: seeding cardiomyocytes in a culture container together with a culture medium; adding a drug to the culture medium in the culture container to bring the drug into contact with the cardiomyocytes; and measuring a heart disease biomarker that is secreted from the cardiomyocytes to assess cardiotoxicity of the drug, wherein at least a culture surface of the culture container is composed of an alicyclic structure-containing polymer, and the culture surface has a surface free energy of 30 to 37 mN/m. The use of a culture container in which at least the culture surface is composed of an alicyclic structure-containing polymer and the surface free energy of the culture surface is 30 to 37 mN/m reduces variations in measured levels of a heart disease biomarker secreted from cardiomyocytes in response to a drug and as such enables highly reproducible measurements.

In the above aspect, it is preferable that the cardiomyocytes are pluripotent stem cell-derived cardiomyocytes. In particular, induced pluripotent stem cell-derived cardiomyocytes are preferred. This is because they pose no ethical problem and stable supply is expected, so that highly accurate measurements can be made even when the measurements are performed over a long period of time.

In the above aspect, it is preferable that the heart disease is heart failure and a biomarker of the heart failure is a natriuretic peptide.

In the above aspect, it is preferable that the alicyclic structure-containing polymer is a hydrogenated norbornene ring-opened polymer. This is because when the culture surface of the culture container is composed of a hydrogenated norbornene ring-opened polymer among other alicyclic structure-containing polymers, variations in measured values of a heart disease biomarker are remarkably reduced.

### (Advantageous Effect)

According to the present disclosure, heart disease biomarkers can be measured with high accuracy.

### BRIEF DESCRIPTION OF THE DRAWINGS

In the accompanying drawings:
FIG. 1 is a graph showing a plot of BNP concentration difference before and after the addition of doxorubicin in Example 1;
FIG. 2 is a graph showing a plot of BNP concentration difference before and after the addition of doxorubicin in Comparative Example 1;
FIG. 3 is a graph showing a plot of BNP concentration difference before and after the addition of doxorubicin in Comparative Example 2; and
FIG. 4 is a graph showing a plot of BNP concentration difference before and after the addition of doxorubicin in Comparative Example 3.

### DETAILED DESCRIPTION

The following provides a detailed description of embodiments of the present disclosure.

The disclosed method is characterized in that, upon measurement of a heart disease biomarker, a culture container is used in which an inner surface of the container to be contacted with a cell (hereinafter "culture surface") has a surface free energy that falls within a specific range, and the culture surface is composed of an alicyclic structure-containing polymer.

The use of such a culture container is considered to allow cells to adhere to the culture surface without imposing an unnecessary stress on the cells to thereby minimize variations in levels of a biomarker secreted from the cells.

Cells used herein are cardiomyocytes, e.g., pluripotent stem cell-derived cardiomyocytes (particularly, induced pluripotent stem cell-derived cardiomyocytes), embryonic stem cell-derived cardiomyocytes, and human-derived cardiomyocytes.

Culture media for culturing the cells described above are not particularly limited as long as cardiomyocytes can be cultured and maintained. Commercially available culture media for culturing cardiomyocytes can be used.

Culture media may be supplemented with additives. Examples of additives include minerals, metals, and vitamin components.

These additives may be used either alone or in combination of two or more types.

Methods of seeding the cells into the culture container are not particularly limited. For example, at least the culture surface is coated with an extracellular matrix or the like, if necessary. Cells suspended in culture medium are then seeded into the culture container by a pipette etc., and if necessary, the container is shaken to evenly distribute the cells throughout the container. The container is then placed in an incubator. Examples of extracellular matrices used herein include natural or synthetic extracellular matrices such as gelatin, fibronectin, vitronectin, and laminin.

Methods of coating the culture surface with an extracellular matrix are similar to common methods of coating a culture container with a cellular substrate. Coating is usually accomplished by methods which include placing the coating agent described above into a culture container, allowing the coating agent to stand in the container at a temperature near the culture temperature usually for 10 minutes to 5 hours, preferably 30 minutes to 2 hours, for contact with the culture surface, and removing the coating agent from the culture surface. Too short contact time results in insufficient coating. On the other hand, because an alicyclic structure-containing polymer constituting the culture surface has low protein adsorption properties and hence does not adsorb protein in multiple layers unlike polystyrene, even if the contact time is prolonged, protein adsorption does not increase. For this reason, it is not necessary to lengthen the contact time beyond the time described above. After removing the coating agent, it is desirable to quickly add a culture medium to prevent drying.

Because the culture surface composed of an alicyclic structure-containing polymer tends to repel an aqueous solution, it is desirable that the amount of the coating agent to be added to the culture container is about 1.5 to 3 times larger than the amount of the coating agent to be added to a general polystyrene cell culture container. Specifically, 0.15 to 0.30 ml of the coating agent is preferably added per 1 cm² of the culture surface.

A novel drug is added into the culture medium for contact with cells to assess whether or not a biomarker is secreted from the cells.

The effectiveness of the above method can be confirmed for example by comparison with the secretion of a heart disease biomarker with a drug known to be cardiotoxic, e.g., doxorubicin, endothelin, or trastuzumab.

Heart disease biomarkers to be measured herein include heart failure biomarkers such as natriuretic peptides, e.g., atrial natriuretic peptide (ANP), brain natriuretic peptide (BNP) (hereinafter "BNP"), C-type natriuretic peptide (CNP), and precursors and degradants thereof; myocardial infarction biomarkers such as troponin T, troponin I, myoglobin, and CK-MB.

Among the foregoing, heart failure biomarkers show a remarkable effect of reducing variations in measured values. Particularly preferred from this viewpoint are brain natriuretic peptides such as BNP and N-terminal pro-B-type natriuretic peptide (NT-proBNP), with BNP being most preferred.

The culture container used herein can be of any shape as long as an alicyclic structure-containing polymer can be used as a material of the culture container. Examples of culture containers used herein include dishes, plates, microchannel chips, bags, tubes, scaffolds, cups, and jar fermenters.

It is only necessary that at least the culture surface of the culture container is composed of an alicyclic structure-containing polymer. For example, in the case of a 96-well plate, it is only necessary that the inner bottom surface of each well is formed of an alicyclic structure-containing polymer. In the case of a bag which is composed of a laminate of films made of different polymer materials, it is only necessary that the inner most layer (interior surface of the bag) is formed of a film made of an alicyclic structure-containing polymer.

Alternatively, the entire culture container may be composed of an alicyclic structure-containing polymer. For example, in the case of a culture dish, a flask, or a plate with a plurality wells, the entire container can be constituted of an alicyclic structure-containing polymer by forming the entire container with the alicyclic structure-containing polymer.

The alicyclic structure-containing polymer is a resin having an alicyclic structure in the main chain and/or side chain. From the viewpoint of mechanical strength, heat resistance and other properties, alicyclic structure-containing polymers with an alicyclic structure in the main chain are preferred, and those having no polar groups are more preferred from the viewpoint of differentiation induction efficiency. A polar group refers to an atom group with polarity. Polar groups include amino, carboxyl, hydroxyl, and acid anhydride groups.

Examples of alicyclic structures include saturated cyclic hydrocarbon (cycloalkane) structures and unsaturated cyclic hydrocarbon (cycloalkene) structures. From the viewpoint of mechanical strength, heat resistance and other properties, cycloalkane structures and cycloalkene structures are preferred, with those having a cycloalkane structure being most preferred.

The number of carbon atoms constituting the alicyclic structure is not particularly limited, but is usually 4 to 30, preferably 5 to 20, and more preferably 5 to 15. When the number of carbon atoms constituting the alicyclic structure falls within this range, mechanical strength, heat resistance, and processability are advantageously highly balanced.

The proportion of a repeat unit having an alicyclic structure in the alicyclic structure-containing polymer may be appropriately selected depending on the purpose of use, but is usually 30% by weight or more, preferably 50% by weight or more, and more preferably 70% by weight or more. If the proportion of a repeat unit having an alicyclic structure in the alicyclic structure-containing polymer is excessively low, heat resistance undesirably lowers. The remainder other than the repeat unit having an alicyclic structure in the alicyclic structure-containing polymer is not particularly limited and is appropriately selected depending on the purpose of use.

Specific examples of alicyclic structure-containing polymers include (1) norbornene polymers, (2) monocyclic cycloolefin polymers, (3) cyclic conjugated diene polymers, (4) vinyl alicyclic hydrocarbon polymers, and hydrogenated products of (1) to (4). Preferred are norbornene polymers and hydrogenated products thereof from the viewpoint of heat resistance, mechanical strength, and other properties.

### (1)Norbornene polymers

Norbornene polymers are obtained by polymerizing a norbornene monomer, a monomer having a norbornene skeleton, and are roughly classified into those obtained by ring-opening polymerization and those obtained by addition polymerization.

Examples of norbornene polymers obtained by ring-opening polymerization include ring-opened polymers of norbornene monomers, ring-opened polymers of norbornene monomers and other monomers copolymerizable with the norbornene monomers by ring-opening polymerization, and hydrogenated products thereof. Examples of norbornene polymers obtained by addition polymerization include addition polymers of norbornene monomers and addition polymers of norbornene monomers and other monomers copolymerizable with the norbornene monomers. Preferred are hydrogenated ring-opened polymers of norbornene monomers from the viewpoint of heat resistance, mechanical strength, and other properties.

Examples of norbornene monomers usable to synthesize norbornene polymers include:
bicyclic monomers such as bicyclo[2.2.1]hepta-2-ene (commonly known as norbornene), 5-methyl-bicyclo[2.2.1]hepta-2-ene, 5,5-dimethyl-bicyclo[2.2.1]hepta-2-ene, 5-ethyl-bicyclo[2.2.1]hepta-2-ene, 5-ethylidene-bicyclo[2.2.1]hepta-2-ene, 5-vinyl-bicyclo[2.2.1]hepta-2-ene, 5-propenylbicyclo[2.2.1]hepta-2-ene,
   5-methoxycarbonyl-bicyclo[2.2.1]hepta-2-ene,
   5-cyanobicyclo[2.2.1]hepta-2-en, and 5-methyl-5-methoxycarbonyl-bicyclo[2.2.1]hepta-2-ene;
tricyclic monomers such as tricyclo[4.3.0^{1,6}.^{2,5}]deca-3,7-diene (commonly known as dicyclopentadiene), 2-methyldicyclopentadiene, 2,3-dimethyldicyclopentadiene, and 2,3-dihydroxydicyclopentadiene; and
tetracyclic monomers such as tetracyclo[4.4.0.1^{2,5}.1^{7,10}]-3-dodecene (tetracyclododecene), tetracyclo[4.4.0.1^{2,5}.1^{7,10}]-3-dodecene, 8-methyltetracyclo[4.4.0.1^{2,5}.1^{7,10}]-3-dodecene,
   8-ethyltetracyclo[4.4.0.1^{2,5}.1^{7,10}]-3-dodecene,
   8-ethylidenetetracyclo[4.4.0.1^{2,5}.1^{7,10}]-3-dodecene,
   8,9-dimethyltetracyclo[4.4.0.1^{2,5}.1^{7,10}]-3-dodecene,
   8-ethyl-9-methyltetracyclo[4.4.0.1^{2,5}. 1^{7,10}]-3-dodecene,
   8-ethylidene-9-methyltetracyclo[4.4.0.1^{2,5}.1^{7,10}]-3-dodecene, 8-methyl-8-carboxymethyltetracyclo[4.4.0.1^{2,5}.1^{7,10}]-3-dodecene, 7,8-benzotricyclo[4.3.0.1^{2,5}]deca-3-ene (commonly known as methanotetrahydrofluorene, also referred to as 1,4-methano-1,4,4a,9a-tetrahydrofluorene),
   1,4-methano-8-methyl-1,4,4a,9a-tetrahydrofluorene,
   1,4-methano-8-chloro-1,4,4 a, 9a-tetrahydrofluorene, and 1,4-methano-8-bromo-1,4,4a,9a-tetrahydrofluorene.

Examples of other monomers copolymerizable with norbornene monomers by ring-opening polymerization include monocyclic cycloolefin monomers such as cyclohexene, cycloheptene, cyclooctene, 1,4-cyclohexadiene, 1,5-cyclooctadiene, 1,5-cyclodecadiene, 1,5,9-cyclododecatriene, and 1,5,9,13-cyclohexadecatetraene.

These monomers may have one or more different substituents. Examples of substituents include alkyl, alkylene, aryl, silyl, alkoxycarbonyl, and alkylidene groups.

Examples of other monomers copolymerizable with norbornene monomers by additional polymerization include C2-C20 α-olefin monomers such as ethylene, propylene, 1-butene, 1-pentene, and 1-hexene; cycloolefin monomers such as cyclobutene, cyclopentene, cyclohexene, cyclooctene, tetracyclo[9.2.1.0^{2,10}.0^{3,8}]tetradeca-3,5,7,12-tetraene (also referred to as 3a,5,6,7a-tetrahydro-4,7-methano-1H-indene); and non-conjugated diene monomers such as 1,4-hexadiene, 4-methyl-1,4-hexadiene, 5-methyl-1,4-hexadiene, and 1,7-octadiene.

Preferred other monomers copolymerizable with norbornene monomers by additional polymerization are α-olefin monomers, with ethylene being more preferred.

These monomers may have one or more different substituents. Examples of substituents include alkyl, alkylene, aryl, silyl, alkoxycarbonyl, and alkylidene groups.

Ring-opened polymers of norbornene monomers or ring-opened polymers of norbornene monomers and other monomers copolymerizable with norbornene monomers by ring-opening polymerization can be obtained by polymerizing monomer components in the presence of a ring-opening polymerization catalyst known in the art. Examples of ring-opening polymerization catalysts usable herein include those consisting of a metal (e.g., ruthenium or osmium) halide and of a nitrate or acetylacetone compound and a reducing agent; and those consisting of a metal (e.g., titanium, zirconium, tungsten, or molybdenum) halide or an acetylacetone compound and of an organoaluminum compound.

A hydrogenated ring-opened polymer of a norbornene monomer can be usually obtained by hydrogenating carbon-carbon unsaturated bonds by adding into a solution of the ring-opened polymer a known hydrogenation catalyst containing a transition metal such as nickel or palladium.

Addition polymers of norbornene monomers or addition polymers of norbornene monomers and other monomers copolymerizable with the norbornene monomers can be obtained by polymerizing monomer components in the presence of a known addition polymerization catalyst. Addition polymerization catalysts usable herein include those consisting of a titanium, zirconium or vanadium compound and of an organoaluminum compound.

### (2) Monocyclic cycloolefin polymers

Monocyclic cycloolefin polymers usable herein include addition polymers of monocyclic cycloolefin monomers such as cyclohexene, cycloheptene, and cyclooctene.

### (3) Cyclic conjugated diene polymers

Cyclic conjugated diene polymers usable herein include polymers obtained by 1,2- or 1,4-addition polymerization of cyclic conjugated diene monomers such as cyclopentadiene and cyclohexadiene, and hydrogenated products thereof.

### (4)Vinyl alicyclic hydrocarbon polymers

Vinyl alicyclic hydrocarbon polymers usable herein include polymers of vinyl alicyclic hydrocarbon monomers such as vinylcyclohexene and vinylcyclohexane, and hydrogenated products thereof; and polymers of vinyl aromatic monomers such as styrene and α-methylstyrene, wherein the aromatic moiety is hydrogenated. Vinyl alicyclic hydrocarbon polymers may be copolymers of these monomers and other monomers copolymerizable with the monomers.

The molecular weight of the alicyclic structure-containing polymer is not particularly limited but is usually 5,000 or more, preferably 5,000 to 500,000, more preferably 8,000 to 200,000, and particularly preferably 10,000 to 100,000, in terms of polyisoprene-equivalent weight-average molecular weight as measured by gel permeation chromatography of the polymer in cyclohexane (or toluene when the polymer is not soluble). When the weight-average molecular weight falls within this range, mechanical strength and processability are advantageously highly balanced.

The glass transition temperature of the alicyclic structure-containing polymer may be appropriately selected depending on the purpose of use, but is usually 50°C to 300°C, and preferably 100°C to 280°C. When the glass-transition temperature is within this range, heat resistance and processability are advantageously highly balanced.

The glass transition temperature of the alicyclic structure-containing polymer herein is measured based on JIS K 7121.

The alicyclic structure-containing polymers may be used alone or in combination of two or more types.

The alicyclic structure-containing polymer may be blended with additives commonly used for thermoplastic resin materials, e.g., soft polymers, antioxidants, ultraviolet absorbers, light stabilizers, near-infrared absorbers, mold release agents, colorants such as dyes or pigments, plasticizers, antistatic agents, and fluorescent brighteners in amounts commonly used.

The alicyclic structure-containing polymer may be mixed with polymers other than soft polymers (hereinafter simply "other polymers"). The amount of other polymers to be mixed with the alicyclic structure-containing polymer is usually 200 parts by mass or less, preferably 150 parts by mass or less, and more preferably 100 parts by mass or less, per 100 parts by mass of the alicyclic structure-containing polymer.

If the proportion of additives and/or other polymers to be blended with the alicyclic structure-containing polymer is too high, cells become difficult to float. To avoid this problem, additives and other polymers are preferably blended so as not to compromise the properties of the alicyclic structure-containing polymer.

Methods of mixing the alicyclic structure-containing polymer with additives and/or other polymers are not particularly limited as long as the additives are sufficiently dispersed in the polymer. The order of blending is also not limited. Examples of blending methods include methods wherein the resin in a molten state is kneaded using a mixer, a uniaxial kneader, a twin-screw kneader, a roll, Bravender, an extruder or the like; and methods in which the components are dissolved in suitable solvent and the solvent is removed by coagulation, casting or direct drying.

When a twin-screw kneader is used, it is often the case that, after kneading, the resin is extruded in a molten state into a strand and the strand is cut into pellets of suitable length using a strand cutter.

Methods of forming a container composed of an alicyclic structure-containing polymer can be freely selected depending on the desired shape of the culture container. Forming methods include injection molding, extrusion molding, cast molding, inflation molding, blow molding, vacuum molding, press molding, compression molding, rotational molding, calender molding, cutting, and spinning. These forming methods can be combined. Where necessary, post-treatment such as stretching can also be performed.

The culture container used herein is preferably sterilized.

Sterilization methods are not particularly limited and can be selected from those commonly used in the medical field according to the shape of the culture container and the cells to be used, e.g., from heat sterilization such as high-pressure steam sterilization and dry heat sterilization; radiation sterilization in which radiation such as γ-rays or electron beams are directed to the container; radio frequency sterilization in which radio frequencies are directed to the container; gas sterilization in which the container is contacted with ethylene oxide gas (EOG) or other gas; and filtration sterilization using a sterilization filter. Gas sterilization methods are preferred because the surface free energy of the culture surface is easily kept in a specific range.

The surface free energy of the culture surface of the culture containers used herein shall fall within the range of 30 to 37 mN/m. In order for the surface free energy of the culture surface to fall within the range described above, it is important not to perform such surface treatment as plasma treatment, which is usually performed to improve the adhesion of cells, on the culture surface. The surface free energy is preferably in the range of 32 to 36 mN/m.

The surface free energy is evaluated by measuring whether several test inks, each given a corresponding surface free energy value in a predetermined range, can be kept adhered on the culture surface for a predetermined period of time. More specifically, when a bead of ink drawn on the culture surface does not change for 2 seconds without becoming droplets, it means that the surface free energy value of the culture surface is equal to or greater than the surface free energy value given to the ink used. The component of ink is a mixture of organic solvents (2-ethoxyethanol, 2-propanol) and a basic dye (formamide); these liquids are mixed in different ratios to produce inks having corresponding surface free energy values.

A specific method of measuring surface free energy is as follows: First, when a bead of ink that is given a surface free energy value of 38 mN/m is drawn on the measurement surface and the bead does not change for 2 seconds without becoming droplets, it means that the surface free energy is equal to or greater than 38 mN/m. In this case, the same measurement is performed using an ink given a surface free energy value of 40 mN/m. If a bead of ink does not change for two seconds without becoming droplets, such measurements are made using inks given higher values until a bead of ink changes to droplets within 2 seconds.

In cases where a bead of ink given a surface free energy value of as low as about 38 mN/m changes into droplets in the measurement, the same measurement is performed using an ink given a value lower than 38 mN/m. For example, when a bead of ink given a surface free energy value of 35 mN/m is drawn on the measurement surface and the bead does not change into droplets within 2 seconds, it can be seen that the surface free energy value is present between the two values (i.e., from 35 to 38 mN/m).

The measurement of heart disease biomarkers is not particularly limited and may be performed using methods known in the art, e.g., using commercially available measurement kits.

### EXAMPLES

Hereinafter, the present disclosure will be specifically described with reference to Examples, which however shall not be construed as limiting the scope of the present disclosure.

### (Example I)

As a culture container a 96-well plate with 96 cylindrical wells each having a bottom area of 0.32 cm² was obtained by injection molding of ZEONOR® 1060R (ZEONOR is a registered trademark in Japan, other countries, or both) (ZEON Corporation, hydrogenated norbornene ring-opened polymer, hereinafter simply "1060R") as an alicyclic structure-containing polymer. The culture container was sterilized with ethylene oxide gas (hereinafter, this culture container is referred to as "1060R 96-well plate"). The surface free energy at the inner bottom surface (cell-contacting surface, or culture surface) of each well of the 1060R 96-well plate was 34 mN/m. The surface free energy was measured using surface energy evaluation inks (Arcotest GmbH).

To each well of the 1066R 96-well plate was added 200 µL of 10 µg/mL human fibronectin (Corning Incorporated, product number: 356008) solution in sterile water and incubated at 37°C for 2 hours to coat the inner bottom surface of each well with human fibronectin. Human fibronectin solution was removed from each well. Each well was seeded with iCell® Cardiomyocytes² (iCell is a registered trademark in Japan, other countries, or both) (Cellular Dynamics International Inc., product number: CMC-100-012-001) at 7.5×10⁴ cells/well using the accompanying thawed culture media for cardiomyocytes. The cells were cultured at 37°C in a 5%CO₂ atmosphere for 4 hours.

Thawed culture media was removed from each well of the plate and 200 µL of maintenance culture media for cardiomyocytes was added to each well and the cells were cultured at 37°C in a 5%CO₂ atmosphere.

Half volume of the maintenance culture media in each well of the plate was exchanged with fresh maintenance media two days after seeding. 20 µL of culture supernatant (pre-addition supernatant sample) was recovered from each well three days after seeding.

Next, doxorubicin (Toronto Research Chemical Inc., product number: D558000) in solution was diluted with dimethyl sulfoxide (DMSO) (NACALAI TESQUE, INC., product number: 13435-35). 20 µL of the diluted doxorubicin solution was added to the 1060R 96-well plate to a final doxorubicin concentration of 10⁻⁶ M in the well. Serial dilutions were further performed such that wells contain final doxorubicin concentrations of 10⁻⁷ M, 10⁻⁸ M, 10⁻⁹ M, 10⁻¹⁰M, 10⁻¹¹ M, 10⁻¹² M, 10⁻¹³M, and 10⁻¹⁴ M. 20 µl of the diluted doxorubicin solution was added to the wells and the cells incubated at 37°C for 16 hours in a 5%CO₂ atmosphere. 20 µL of supernatant (post-addition supernatant sample) was recovered from each well loaded with the doxorubicin solution.

Supernatant samples before and after doxorubicin addition were measured for BNP level using Human BNP ELISA Kit (RayBiotech, product number: ELH-BNP).

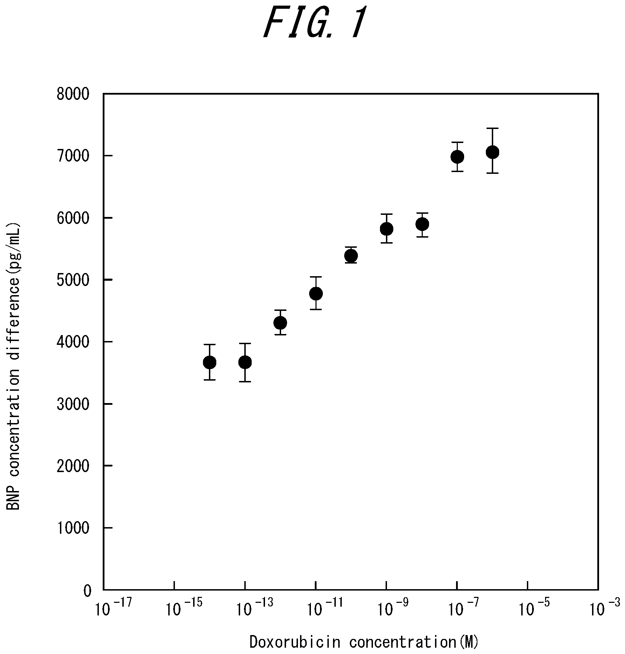

FIG. 1 is a graph showing a plot of cell-secreted BNP concentration difference before and after addition of doxorubicin. BNP concentration differences were plotted with error bars representing standard deviations from the mean (n=3) for each doxorubicin concentration. The results shown in the graph depicted in FIG. 1 reveal that variations in cell-secreted BNP concentration differences before and after the addition of doxorubicin are extremely small. In addition, a correlation between doxorubicin concentration and BNP concentration difference (BNP concentration difference increases with increasing doxorubicin concentration) can be read from the graph of FIG. 1.

### (Comparative Example 1)

Cell culture, addition of doxorubicin, and recovery of supernatants were performed as in Example 1 except that the 1060R 96-well plate used in Example 1 was replaced by a 1060R 96-well plate (surface free energy value: 52 mN/m as measured using surface energy value evaluation inks as described above) in which the inner bottom surface and the inner wall of each well were subjected to plasma treatment at 100V for 60 seconds using a plasma treatment device (Izumi Industry Corporation, product number IP-200), and BNP concentrations before and after addition of doxorubicin were measured.

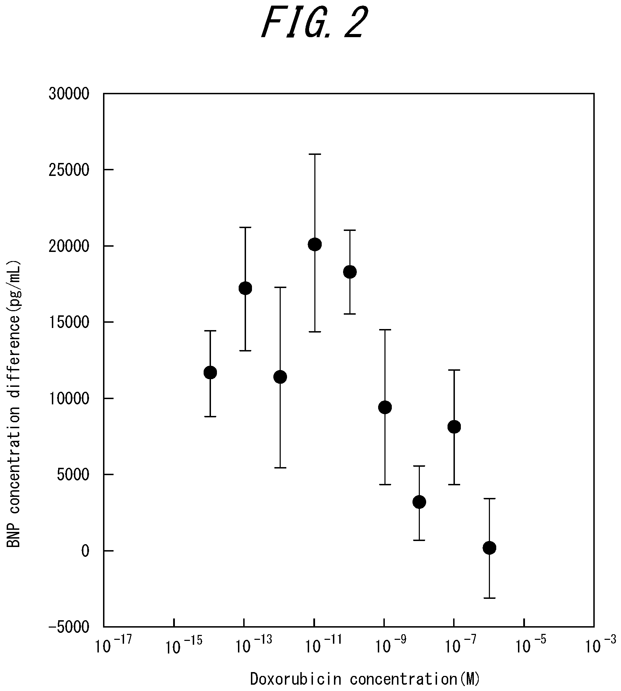

FIG. 2 is a graph showing a plot of cell-secreted BNP concentration difference before and after addition of doxorubicin. BNP concentration differences were plotted with error bars representing standard deviations from the mean (n=3) for each doxorubicin concentration. From the results of FIG. 2, it can be understood that the use of a well plate in which the surface free energy of the culture surface falls outside a specific range due to surface treatment increases variations in measured values of the BNP concentration difference and hence the measurement accuracy is remarkably lowered. Further, due to lowered measurement accuracy, a correlation between doxorubicin concentration and BNP concentration difference cannot be read from the graph.

### (Comparative Example 2)

Cell culture, addition of doxorubicin, and recovery of supernatants were performed as in Example 1 except that the 1060R 96-well plate used in Example 1 was replaced by a polystyrene 96-well plate (Falcon® (Falcon is a registered trademark in Japan, other countries, or both), Corning Incorporated, product number: 353916; surface free energy: 43 mN/m), and BNP concentrations before and after addition of doxorubicin were measured.

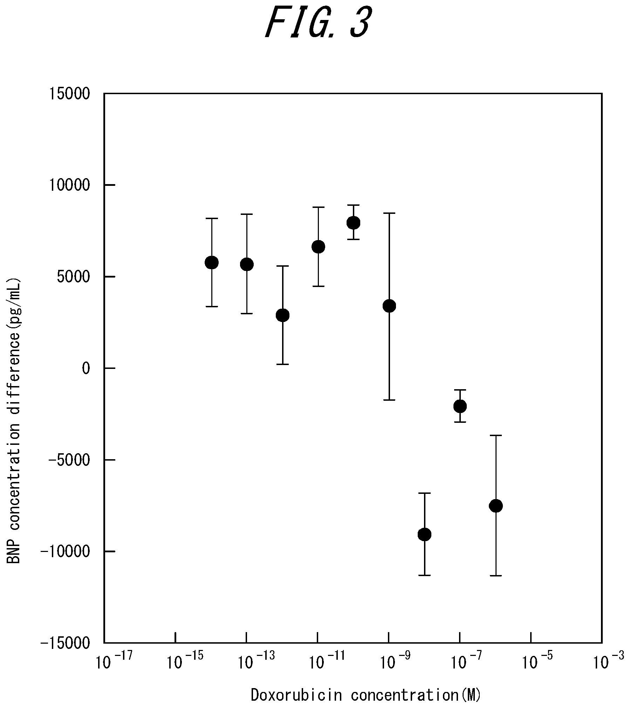

FIG. 3 is a graph showing a plot of cell-secreted BNP concentration difference before and after addition of doxorubicin. BNP concentration differences were plotted with error bars representing standard deviations from the mean (n=3) for each doxorubicin concentration. BNP concentration differences were plotted with error bars representing standard deviations from the mean (n=3) for each doxorubicin concentration. From the results shown in FIG. 3, it can be seen that the use of a well plate in which the culture surface is not composed of an alicyclic structure-containing polymer and has a surface free energy of higher than 37 mN/m, like a polystyrene well plate, increases variations in BNP concentration differences and hence the measurement accuracy is remarkably lowered. Further, due to lowered measurement accuracy, a correlation between doxorubicin concentration and BNP concentration cannot be read.

### (Comparative Example 3)

Cell culture, addition of doxorubicin, and recovery of supernatants were performed as in Example 1 except that the 1060R 96-well plate used in Example 1 was replaced by a polystyrene 96-well plate for tissue culture (Falcon® (Falcon is a registered trademark in Japan, other countries, or both), Corning Incorporated, product number: 351172; surface free energy: 37 mN/m), and BNP concentrations before and after addition of doxorubicin were measured.

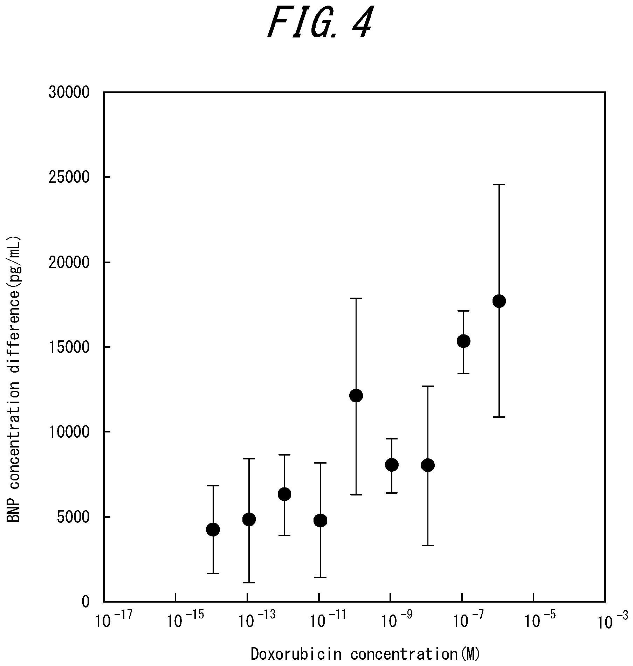

FIG. 4 is a graph showing a plot of cell-secreted BNP concentration difference before and after addition of doxorubicin. BNP concentration differences were plotted with error bars representing standard deviations from the mean (n=3) for each doxorubicin concentration. BNP concentration differences were plotted with error bars representing standard deviations from the mean (n=3) for each doxorubicin concentration. From the results of FIG. 4, it can be seen that the use of a well plate in which the culture surface is not composed of an alicyclic structure-containing polymer, even when the surface free energy value of the culture surface falls within a specific range, increases variations in measured values of the BNP concentration difference and hence the measurement accuracy is remarkably lowered.

From the results of FIGS. 1 to 4, it can be seen that the use of a well plate in which the surface free energy value of the culture surface falls within a specific range and the culture surface is composed of an alicyclic structure-containing polymer remarkably reduces variations in measured values of the BNP concentration and thus enables highly reproducible measurement.

It should be noted that when the surface free energy of the culture surface is lower than 30 mN/m, the culture surface becomes highly hydrophobic and as such protein adsorption due to hydrophobic interaction is more promoted. Accordingly, in this case, variations in BNP concentration differences increase as in Comparative Example 2 in which the culture surface is subjected to surface treatment, resulting in the measurement accuracy being remarkably lowered.

Further, even for a biomarker other than BNP, as long as it is a stress biomarker as described above, it is possible to remarkably reduce variations in measured values as with BNP described above by allowing cells to adhere to the culture container with a minimum stress.

### INDUSTRIAL APPLICABILITY

According to the disclosed cardiotoxicity assessment method, it is possible to measure a heart disease biomarker with high accuracy and therefore to perform accurate cardiotoxicity assessment.

This allows accurate measurement of the toxicity of compounds already known to have cardiotoxicity. Thus, the disclosed assessment method can be utilized to assess the toxicity of other new drugs while using, for example, doxorubicin as a positive control and eventually to determine the dosage of the drugs for maximizing efficacy while reducing toxicity.

## Claims

1. A cardiotoxicity assessment method comprising:
seeding cardiomyocytes in a culture container together with a culture medium;
adding a drug to the culture medium in the culture container to bring the drug into contact with the cardiomyocytes; and
measuring a heart disease biomarker secreted from the cardiomyocytes to assess cardiotoxicity of the drug,
wherein at least a culture surface of the culture container is composed of an alicyclic structure-containing polymer, and the culture surface has a surface free energy of 30 to 37 mN/m.

2. The cardiotoxicity assessment method according to claim 1, wherein the cardiomyocytes are pluripotent stem cell-derived cardiomyocytes.

3. The cardiotoxicity assessment method according to claim 1 or 2, wherein the heart disease is heart failure and a biomarker of the heart failure is a natriuretic peptide.

4. The cardiotoxicity assessment method according to any one of claims 1 to 3, wherein the alicyclic structure-containing polymer is a hydrogenated norbornene ring-opened polymer.
